# FASCICULE DE BREVET EUROPEEN

(11) **EP 3 139 905 B1**
(45) Date de publication et mention de la délivrance du brevet: **22.04.2020**
(21) Numéro de dépôt: 15723273.7
(22) Date de dépôt: 28.04.2015
(51) Int. Cl.: A61K 9/16, A61K 9/20

(54) **CO-GRANULES DE GOMME DE XANTHANE ET DE GOMME D'ACACIA**
CO-GRANULATE VON XANTHAN- UND AKAZIENGUMMI
CO-GRANULES OF XANTHAN GUM AND ACACIA GUM

(30) Priorité: 05.05.2014 FR 1454061
(43) Date de publication de la demande: 15.03.2017
(73) Titulaire: Société d'Exploitation de Produits pour les Industries Chimiques SEPPIC, 75321 Paris cedex 07 (FR)
(72) Inventeur: LEFEBVRE, Sandra, 81100 Castres (FR)
(74) Mandataire: Laigneau, Amandine
(86) Numéro de dépôt international: PCT/FR2015/051149
(87) Numéro de publication internationale: WO 2015/170038

(56) Documents cités:
- EP-A2- 0 360 562
- FR-A1- 2 983 409
- US-A1- 2009 175 941
- US-B1- 6 221 393

## Description

L'invention a pour objet des co-granulés de gomme de xanthane et de gomme d'acacia, possédant un diamètre moyen compris entre 50 µm et 1000 µm, caractérisé en ce que le rapport massique entre la gomme de xanthane et la gomme d'acacia varie de 4/1 à 1/1, utilisés pour la préparation de compositions pharmaceutiques, de compositions de compléments alimentaires ou de compositions alimentaires sous forme de comprimés à libération prolongée (dénommées également à effet retard) et leur procédé de préparation.

Lesdites compositions pharmaceutiques, de compléments alimentaires ou alimentaires contiennent un principe pharmacologiquement actif et/ou un agent nutritionnel, et sont par exemple utilisées quand on souhaite administrer un médicament à un patient pendant une durée prolongée sans exiger du patient de prendre des doses répétées à intervalles réduits.

Le « Handbook of pharmaceutical excipients 7th Edition, 2012 » décrit les différentes applications possibles de la gomme de xanthane. Cette dernière est largement utilisée comme agent épaississant. Des associations synergétiques ont été mises en évidence entre la gomme de xanthane et des galactomannanes (gomme de guar, gomme de caroube, gomme de casse...) permettant d'obtenir des gels de très haute viscosité.

La gomme de xanthane est un excipient connu dans le domaine pharmaceutique et de la nutrition pour son utilisation dans la formulation de comprimés à libération prolongée (dénommés également matrices hydrophiles). Dans le livre «Oral Controlled Release Formulation Design and Drug Delivery: Theory to Practice. Edited by Hong Wen - Kinam Park. Sept 2010 », une définition d'un comprimé à libération prolongée est donnée.

Il s'agit d'un comprimé formulé de telle manière que le principe actif est relargué sur une période de temps définie après exposition à un milieu aqueux ou après administration par voie orale. Un tel comprimé est de manière traditionnelle constituée d'un principe actif ou d'un mélange de principes actifs et/ou d'un agent nutritionnel ou d'un mélange d'agents nutritionnels, d'au moins un polymère épaississant, d'additifs technologiques, comme par exemple un agent diluant, un agent lubrifiant, un agent d'écoulement, et fabriquée par compression directe du mélange de l'ensemble de ses constituants.

La compression directe est un procédé de mise en forme galénique particulièrement intéressant car il fait intervenir un nombre limité d'opérations et de constituants et par conséquent nécessite pour sa mise en œuvre des installations moins onéreuses que pour un procédé de préparation de comprimés par granulation humide.

A travers les publications : "Comparative study on xanthan gum and hydroxypropylmethylcellulose as matrices for controlled-release drug delivery I, Compaction and in vitro drug release behavior", International Journal of Pharmaceutics 129 (1996) 233-241*; et* "Swelling and drug release behaviour of xanthan gum matrix tablets", International Journal of Pharmaceutics 120 (1995) 63-72, Mohammad Mahiuddin Takudar de la Faculté de Louvain a largement étudié la formulation de comprimés à libération prolongée en utilisant la gomme de xanthane comme polymère épaississant. Des associations gomme de xanthane/ galactomannanes ont également été testées par différents scientifiques.

Les gommes hydrophiles naturelles ou synthétiques qui sont des polysaccharides de haut poids moléculaire sont connues en tant qu'excipients pharmaceutiques mais toutes les gommes ne sont pas utilisables dans des compositions à effet prolongé dans les procédés de compression à sec pour préparer des comprimés.

Ces polysaccharides sont soit d'origine microbienne, et sont alors obtenus par fermentation d'un hydrate de carbone assimilable par un microorganisme approprié (exemple : la gomme xanthane obtenue à partir de Xanthomonas campesiris), soit d'origine naturelle comme par exemple les gommes de guar et de caroube.

Les gommes de xanthane sont également des excipients connus pour leur utilisation possible dans le domaine pharmaceutique, notamment pour la constitution de matrices destinées à la préparation de formes à libération contrôlée. Cependant les gommes hydrophiles en général et la gomme de xanthane en particulier ne sont en général pas utilisées à l'état de pré granulé.

EP0805676 décrit l'utilisation de gomme de xanthane surtout à forte teneur (généralement à une teneur d'environ 30% massique pour 100% de la masse totale du comprimé) conduisant à une matrice hydrophile, qui présente néanmoins de nombreux défauts tels que des mauvaises propriétés mécaniques...

Un but de la présente invention est de préparer un comprimé à effet prolongé pouvant être fabriqué sans difficulté par compression directe et présentant de bonnes propriétés de libération du principe actif et de meilleures propriétés mécaniques que ceux décrits dans l'état de la technique par exemple dans le brevet EP0805676.

C'est pourquoi la présente invention a pour objet un co-granulé de gomme de xanthane et de gomme d'acacia, caractérisé en ce que le rapport massique entre la gomme de xanthane et la gomme d'acacia varie de 4/1 à 1/1, et possédant un diamètre moyen compris entre 50 µm et 1000 µm.

Dans les produits objets de la présente invention, par gomme de xanthane, on désigne un hétéropolymère d'oses et d'acides uroniques, obtenu par la fermentation aérobie des bactéries du genre *Xanthomonas campestris.* Sa structure est constituée d'une chaîne principale d'unités β-D-glucose reliées entre elles par les carbones 1 et 4.

On compte un triholoside branché toutes les deux unités de glucose dans la chaîne principale, de façon alternative régulière ; chaque branchement consistant en un triholoside composé de deux mannoses et d'un acide glucuronique, du type : β-D-Man*p*-(1 → 4)-β-D-GlcA*p*-(1 → 2)-α-D-Man*p*-(1 → 3).

Les gommes xanthane sont disponibles sous forme d'un sel de sodium, de potassium ou de calcium, et elles se caractérisent par un poids moléculaire compris entre 1.000.000 et 50.000.000.

Les gommes de xanthane sont représentées par exemple par les produits vendus sous le nom commercial Rhodicare™ par la société Rhodia Chimie et sous le nom de marque Keltrol™ CG-T par la société CP-KELCO.

Dans les produits objets de la présente invention, par gomme d'acacia, on désigne un hétéropolymère d'oses et d'acides uroniques, complexe branché dont la chaîne principale consiste en des unités de β-D-galactose reliées entre elles par les carbones 1 et 3.

Les chaînes branchées à la chaîne principale sont constituées d'unités de β-D-galactose reliées entre elles par les carbones 1 et 6, portant également des unités de α-arabinose, et dans de moindres proportions des unités β-glucoronosyle. A la fois la chaîne principale et les chaînes pendantes contiennent des unités α-L-arabinosyles, α-L-rhamnopyranosyles, β-D-glucuronopyranosyles et 4-O-méthyl-β-D-glucuronopyranosyles.

La gomme d'acacia est aussi désignée sous l'appellation « gomme arabique » et constitue une exsudat de sève descendante solidifié, amalgamé naturellement ou par incision sur le tronc et au pied d'arbres de la famille des acacias.

La gomme d'acacia mise en œuvre dans la présente invention est représentée par exemple par le produit vendu sous la dénomination commerciale Efficacia™ M par la société Colloïdes Naturels International.

Selon un aspect particulier, la présente invention a pour objet :
- Un co-granulé tel que défini ci-dessus, possédant un diamètre moyen compris entre 100 µm et 300 µm, de préférence égal à environ 150 µm.
- Un co-granulé tel que défini ci-dessus, caractérisé en ce qu'au plus 4% massique des particules du co-granulé ont un diamètre moyen supérieur à 500 µm et de 65% à 90% massique des particules du co-granulé ont un diamètre moyen supérieur à 80 µm. méthode (Pharmacopée Européenne 8ème Edition 2014 (méthode 2.9.38.)).
- Un co-granulé tel que défini ci-dessus, ayant une masse volumique comprise entre 0,3 mg/mL et 0,8 mg/mL et de préférence comprise entre 0,35 mg/mL et 0,7 mg/mL (Pharmacopée Européenne 8^{ème} Edition 2014 (méthode 2.9.34.)).
- Un co-granulé tel que défini ci-dessus, caractérisé en ce le rapport massique entre la gomme de xanthane et la gomme d'acacia varie de 4/1 à 1/1 et de préférence est égal à 3/1.

La présente invention a aussi pour objet :
- Une composition comprenant au moins un comprimé contenant au moins un principe actif et/ou au moins un agent nutritionnel, caractérisé en ce que ledit comprimé comprend de 15% à 50% massique de co-granulés, et de préférence de 20% à 40%, tels que définis ci-dessus.
- Une composition telle que définie ci-dessus, caractérisée en ce que le comprimé comporte en outre au moins un excipient choisi parmi au moins : un diluant, un agent lubrifiant, un agent de cohésion.
- Une composition telle que définie ci-dessus, caractérisée en ce qu'elle comprend pour 100% de sa masse de 20% à 35% massique de co-granulés tels que définis précédemment, de 0,1% à 60% massique d'un principe actif et/ou d'un agent nutritionnel, de 10% à 50% massique d'agent diluant ou de cohésion, de 0,5% à 3% massique d'agent lubrifiant.
- Une composition telle que définie ci-dessus, caractérisée en ce qu'elle est une composition pharmaceutique, de complément alimentaire ou alimentaire ayant un effet à libération prolongée.

La présente invention a aussi pour objet :
- Un procédé de préparation d'une composition telle que définie à l'une des revendications ci-dessus, comprenant au moins une étape de compression directe d'une mélange comprenant de 15% et 50% en poids de co-granulés, tels que définis précédemment, de préférence de 20% à 40%, et d'au moins un principe actif et/ou d'au moins un agent nutritionnel.
- Un procédé tel que défini ci-dessus, dans le quel ladite composition comprend en outre au moins un excipient choisi parmi au moins : un diluant, un agent lubrifiant, un agent de cohésion.

La présente invention a aussi pour objet :
L'utilisation d'au moins un co-granulé tel que défini à l'une précédemment, pour la fabrication d'une composition pharmaceutique, d'une composition de complément alimentaire ou alimentaire telle que définie à l'une des revendications ci-dessus.

Les comprimés de la composition objet de la présente invention sont caractérisés par :
- Une résistance à la rupture la plus élevée possible (idéalement > 90N) ;
- Une friabilité la plus faible possible (idéalement < 0.5%) ;
- Une libération progressive du principe actif après exposition à un milieu aqueux (temps de désagrégation du comprimé > 1h).

Les comprimés à libération prolongée réalisés avec les co-granulés comprenant de la gomme de xanthane et de la gomme d'acacia présentent des propriétés mécaniques nettement supérieures aux comprimés à libération prolongée fabriqués avec la seule gomme de xanthane sous forme de poudre ou de la seule gomme de xanthane granulée (cf Exemple 2).

Le co-granulé de la présente invention contiendra pour 100% de sa masse au minimum 20% massique de gomme d'acacia et donc au maximum 80% massique de gomme de xanthane.

Cette teneur minimale de 20% massique de gomme d'acacia permet de garantir que les comprimés à libération prolongée fabriqués présentent une résistance à la rupture élevée (cf Exemple 1).

Le co-granulé de la présente invention contiendra pour 100% de sa masse au minimum 50% massique de gomme de xanthane. Cette teneur minimale de 50% massique de gomme de xanthane permet de garantir un relarguage progressif du principe actif après exposition à un milieu aqueux (cf exemple 3).

Dans le cadre de la présente invention, le terme « co-granulé » signifie non pas un simple mélange d'au moins deux composés mais une association dans laquelle les composés sont intimement liés. Autrement dit, les constituants essentiels des co-granulés sont au moins physiquement liés entre eux.

La préparation des co-granulés de la présente invention peut se faire par tous les moyens disponibles de mise en contact d'une solution de gomme d'acacia et des particules solides de gomme xanthane (granulateur, lit d'air fluidisé, atomiseur, tambour tournant, tours d'atomisation...).

Il existe de nombreux procédés décrits dans la littérature pour préparer des co-granulés.

Parmi ces procédés connus, on peut citer US 3 551133 décrivant un procédé de préparation de co-granulés de gommes de xanthane et de caroube à partir de la pulvérisation d'une solution aqueuse d'un mélange de poudre des deux gommes sur des granulateurs à plateau incliné ou à disque.

GB-2086204 décrit un procédé du même type.

Selon EP-206 368, on pulvérise une solution de gommes sur un lit fluidisé de gommes.

Le procédé préféré de granulation est, selon l'invention, le procédé selon lequel on pulvérise sur la gomme de xanthane une solution de gomme d'acacia, en lit fluidisé à l'aide d'un courant gazeux et on obtient les granules par séchage.

Cependant le comprimé à libération prolongée peut comporter en outre un ou plusieurs excipients pharmaceutiquement et/ou nutritionnellement acceptables, plus particulièrement des agents diluants, des agents de cohésion, des agents lubrifiants.

Parmi les agents diluants que l'on peut associer dans le comprimé à libération prolongée, on peut citer le lactose, le sucrose, le mannitol, le xylitol, l'isomalt, l'hydrogénophosphate de calcium, la cellulose microcristalline, les amidons et plus particulièrement les amidons pré-gélatinisés,les carbonates de calcium et de magnésium...

Parmi les agents lubrifiants que l'on peut associer dans le comprimé à libération prolongée, on peut citer le stéarate de magnésium, le talc, le Stearyl Fumarate de sodium, les huiles végétales hydrogénées, l'acide stéarique...

La présente invention peut être utilisée pour la compression directe d'agents nutritionnels et de principes actifs appartenant à toutes les classes de médicaments destinés à l'administration par voie orale.

Parmi les principes actifs utilisés dans des compositions selon la présente invention on peut citer les anti-rhumatismaux et anti-inflammatoires non stéroïdiens (ketoprofène, ibuprofène, flurbiprofène, indométaclne, phénylbutazone, allopurinol...), les analgésiques (paracétamol, phénacétine, aspirine...). les antitussifs (codéine, codéthyline, alimemazine...), les stérols (hydrocortisone, cortisone, progestérone, testostérone, triamcinolone, dexamethazone, betamethazone, paramethazone, fluocinolone, beclomethazone...), les barbituriques (barbital, allobarbital, phenobarbital, pentobarbital, amobarbital...), les antimicrobiens (pefloxacine, sparfloxacine, et dérivés de la classe des quinolones, tetracylines, synergistines, metronidazole...), les médicaments destines au traitement des allergies, les anti-asthmatiques, les vitamines (vitamine A, vitamine E, vitamine du groupe D, vitamine K), les antispasmodiques et anti sécrétoires (omeprazole), les cardiovasculaires et les vasodilatateurs cérébraux (quinacainol, oxprenolol, propanolol, nicergotine...), les protecteurs cérébraux, les protecteurs hépatiques les agents thérapeutiques du tractus gastro intestinal, les vaccins, les antihypertenseurs et les cardioprotecteurs tels que les bétabloquants et les dérivés nitrés. Parmi les agents nutritionnels utilisés dans des compositions selon la présente invention on peut citer les sels minéraux (calcium, magnésium, fer, zinc, sodium, potassium, cuivre, manganèse...), les extraits de plantes (bardane, bourrache, mélisse, houblon, lavande, ortie blanche, queue de cerise, reine des prés, Ginseng, guarana, gingembre, Passiflore, valériane, aubépine, tilleul, verveine, ...), les acides gras (oméga 3, oméga 6).

Selon l'invention, l'opération de compression consécutive au mélange des excipients et du principe actif ou de l'agent nutritionnel est généralement mise en œuvre sous une force pouvant aller de 6 a 20 KN (mesure au niveau du galet de compression) et de préférence de l'ordre de 8 a 12 kN.

Cette opération de compression est, de préférence, précédée dune pré compression sous une force pouvant aller de 0,5 a 2,5 kN.

De grandes vitesses de compression peuvent être atteintes grâce au procédé selon l'invention, sans pour autant altérer la qualité des comprimés. Il est notamment possible d'atteindre des vitesses supérieures à 150000 comprimés par heure, sans entraîner de clivage.

Il est entendu que les comprimés obtenus selon l'invention peuvent éventuellement être pelliculés selon les méthodes habituelles. L'opération de pelliculage est facilitée par le fait qu'aucun clivage n'intervient au cours de l'opération.

Dans ce qui suit ou ce qui précède, sauf indications contraires, les pourcentages et parties sont en poids.

Les exemples suivants illustrent l'invention sans toutefois la limiter.

### Mode opératoire de préparation de comprimés:

On mélange au préalable dans un mélangeur de type Turbula T2C, 700g du mélange de poudre constitué par un principe actif, en l'occurrence la caféine synthétique et les différents constituants de la matrice, à savoir le cogranulé gomme de xanthane-gomme d'acacia et éventuellement les autres excipients, comme par exemple le phosphate di calcique. Le mélange de poudre contient aussi l'agent lubrifiant à savoir le stéarate de magnésium dosé à 1% en poids dans ledit mélange.

La compression est mise en œuvre sous une force de 8,5 kN (mesurée au niveau du galet de compression) en utilisant une machine rotative de type PICOLA NOVA qui permet de réaliser, à partir du mélange de poudre, 1000 comprimés de 500 mg chacun.

### Exemples :

### Exemple 1 :

Des co-granulés contenant des proportions massiques différentes de gomme de xanthane et gomme acacia ont été fabriqués.

Des comprimés à libération prolongée ont été préparés avec ces co-granulés selon la composition suivante :

| Composition du mélange : | Formule (%): |
|---|---|
| Co-granulés | *35%* |
| Caféine synthétique | *20%* |
| MCC (Méthyl carboxy cellulose) Vivapur (JRS) | *33%* |
| Calcium Hydrogen Phosphate dihydrate Ph Eur | *11%* |
| Emcompress (JRS) | |
| Stéarate de Mg | *1%* |

La résistance à la rupture des comprimés a été contrôlée en suivant les méthodes de la Pharmacopée Européenne 8ème Edition 2014 (méthode 2.9.8.). Seuls les co-granulés possédant un rapport massique entre la gomme de xanthane et la gomme d'acacia variant de 4/1 à 1/1, font partis de l'objet des revendications.

| Composition massique co-granulé : | | | | |
|---|---|---|---|---|
| % xanthane | 100% | 90% | 80 | 75 |
| % acacia | 0% | 10% | 20 | 25 |
| Résistance à la rupture des comprimés (N) | 72 | 78 | 91 | 103 |

Un minimum de 20% de gomme d'acacia dans le co-granulé permet d'obtenir des comprimés dont la résistance à la rupture est supérieure à 90N.

### Exemple 2 :

Des comprimés à libération prolongée avec 2 types de gommes disponibles dans le commerce ont été formulés :
- Gomme de xanthane poudre (diamètre moyen des particules : 74µm)
- Gomme de xanthane granulée (diamètre moyen des particules : 185µm)

En parallèle, des comprimés à libération prolongée ont été fabriqués avec les co-granulés de gomme de xanthane et de gomme d'acacia (75/25 p/p) de diamètre moyen : 150µm.

La même formule de compression a été utilisée :

| Composition du mélange : | Formule (%): |
|---|---|
| Co-granulés xanthane-acacia, ou | *35%* |
| gomme xanthane poudre, ou | |
| gomme xanthane granulée | |
| Caféine synthétique | *20%* |
| MCC | *33%* |
| Vivapur (JRS) | |
| Calcium Hydrogen Phosphate dihydrate Ph Européenne Emcompress(JRS) | *11%* |
| Stéarate de Mg | *1%* |

Les mélanges ont été comprimés sur la presse rotative PICOLA NOVA avec les paramètres suivants :
- 8 poinçons de diamètre 11mm
- masse visée : 500 mg / comprimé

Différentes forces de compression ont été testées.

La résistance à la rupture des comprimés et leur friabilité ont été contrôlées en suivant les méthodes de la Pharmacopée Européenne 8ème Edition 2014 (méthodes 2.9.7 et 2.9.8.).

Les résultats suivants ont été obtenus avec la gomme de xanthane poudre :

| Force de compression (kN) | Résistance à la rupture des comprimés (N) | % friabilité des comprimés |
|---|---|---|
| 11 | 43 | 1.4 |
| 13 | 57 | 1.2 |
| 18 | 73 | 0.7 |
| 23 | 82 | 0.6 |
| 25 | 87 | 0.6 |
| 27 | 92 | 0.6 |

Résultats avec la gomme de xanthane granulée :

| Force de compression (kN) | Résistance à la rupture des comprimés (N) | % friabilité des comprimés |
|---|---|---|
| 9 | 54 | 1.1 |
| 13 | 63 | 1.0 |
| 15 | 75 | 0.8 |
| 20 | 80 | 0.8 |
| 23 | 88 | 0.7 |
| 26 | 92 | 0.8 |
| 29 | 92 | 0.8 |

Résultats avec le co-granulé xanthane/ acacia (75/25) :

| Force de compression (kN) | Résistance à la rupture des comprimés (N) | % friabilité des comprimés |
|---|---|---|
| 11 | 103 | 0.6 |
| 14 | 116 | 0.4 |
| 22 | 142 | 0.4 |
| 29 | 162 | 0.4 |

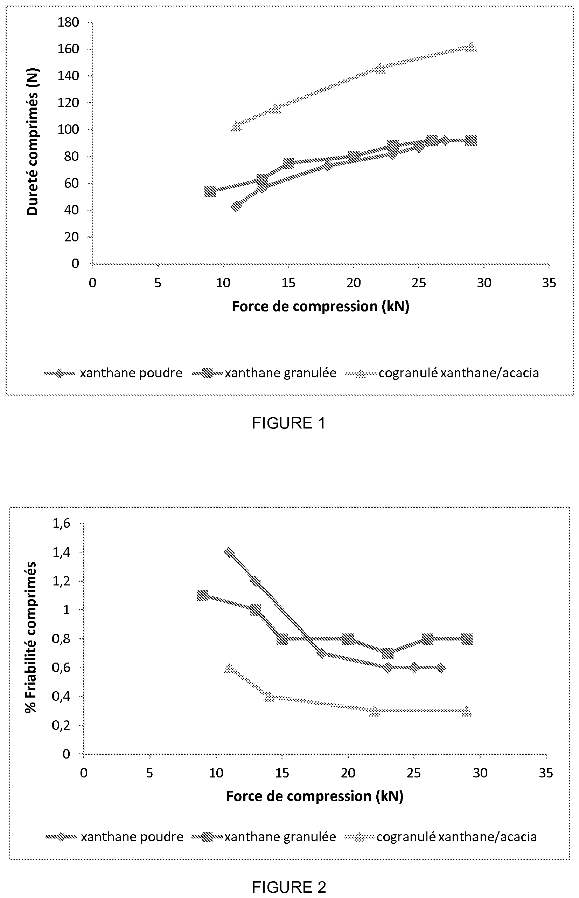

Les figures 1 (dureté des comprimés en fonction de la force de compression) et 2 (friabilité des comprimés en fonction de la force de compression) illustrent qu'à force de compression égale, les comprimés fabriqués avec le co-granulé xanthane/ acacia présentent des propriétés mécaniques supérieures aux comprimés sur gomme xanthane : une résistance à la rupture plus élevée et une friabilité plus faible.

Ceci est valable quelle que soit la granulométrie de la gomme de xanthane témoin (poudre ou granulée).

### Exemple 3 :

Des co-granulés contenant des proportions massiques différentes de gomme de xanthane et gomme acacia ont été fabriqués. Des comprimés ont été préparés avec ces co-granulés selon la composition suivante :

| Composition du mélange : | Formule (%): |
|---|---|
| Co-granulés | *35%* |
| Caféine synthétique | *20%* |
| MCC Vivapur (JRS) | *33%* |
| Calcium Hydrogen Phosphate dihydrate Ph Européenne Emcompress (JRS) | *11%* |
| Stéarate de Mg | *1%* |

Le temps de désagrégation des comprimés a été contrôlé en suivant les méthodes de la Pharmacopée Européenne 8ème Edition 2014 (méthode 2.9.1.). Seuls les co-granulés possédant un rapport massique entre la gomme de xanthane et la gomme d'acacia variant de 4/1 à 1/1, font partis de l'objet des revendications.

| Composition massique co-granulé | | | |
|---|---|---|---|
| % xanthane | 100% | 75 | 50 |
| % acacia | 0% | 25 | 50 |
| Temps de désagrégation des comprimés (N) | >7 heures | 6.5 heures | 90 minutes |

Un minimum de 50% de gomme de xanthane dans le co-granulé permet d'obtenir des comprimés à libération prolongée.

## Revendications

1. Co-granulé de gomme de xanthane et de gomme d'acacia possédant un diamètre moyen compris entre 50 µm et 1000 µm, **caractérisé en ce que** le rapport massique entre la gomme de xanthane et la gomme d'acacia varie de 4/1 à 1/1.

2. Co-granulé selon la revendication 1, possédant un diamètre moyen compris entre 100 µm et 300 µm, de préférence égal à environ 150 µm.

3. Co-granulé selon l'une des revendications 1 ou 2, **caractérisé en ce qu'**au plus 4% massique des particules du co-granulé ont un diamètre moyen supérieur à 500 µm et de 65% à 90% massique des particules du co-granulé ont un diamètre moyen supérieur à 80 µm.

4. Co-granulé selon l'une des revendications précédentes ayant une masse volumique comprise entre 0,3 mg/mL et 0,8 mg/mL et de préférence comprise entre 0,35 mg/mL et 0,7 mg/mL.

5. Co-granulé selon l'une des revendications précédentes, caractérisé en ce le rapport massique entre la gomme de xanthane et la gomme d'acacia est égal à 3/1.

6. Composition comprenant au moins un comprimé contenant au moins un principe actif et/ou au moins un agent nutritionnel, **caractérisé en ce que** ledit comprimé comprend de 15% à 50% massique de co-granulés, et de préférence de 20% à 40%, tels que définis à l'une des revendications 1 à 5.

7. Composition selon la revendication précédente **caractérisé en ce que** le comprimé comporte en outre au moins un excipient choisi parmi au moins : un diluant, un agent lubrifiant, un agent de cohésion.

8. Composition selon la revendication 7 **caractérisée en ce qu'**elle comprend pour 100% de sa masse de 20% à 35% massique de co-granulés tels que définis à l'une des revendications 1 à 5, de 0,1% à 60% massique d'un principe actif et/ou d'un agent nutritionnel, de 10% à 50% massique d'agent diluant ou de cohésion, de 0,5% à 3% massique d'agent lubrifiant.

9. Composition selon l'une des revendications 6 à 8 **caractérisée en ce qu'**elle est une composition pharmaceutique, de complément alimentaire ou alimentaire ayant un effet à libération prolongée.

10. Procédé de préparation d'une composition telle que définie à l'une des revendications 6 à 9 comprenant au moins une étape de compression directe d'une mélange comprenant de 15% et 40% en poids de co-granulés tels que définis à l'une des revendications 1 à 5, de préférence de 20% à 40%, et d'au moins un principe actif et/ou d'au moins un agent nutritionnel.

11. Procédé selon la revendication précédente dans le quel ladite composition comprend en outre au moins un excipient choisi parmi au moins : un diluant, un agent lubrifiant, un agent de cohésion.

12. Utilisation d'au moins un co-granulé tel que défini à l'une des revendications 1 à 5 pour la fabrication d'une composition pharmaceutique, d'une composition de complément alimentaire ou alimentaire telle que définie à l'une des revendications 6 à 9.

## Patentansprüche

1. Co-Granulat aus Xanthangummi und aus Akaziengummi, das einen mittleren Durchmesser zwischen 50 µm und 1000 µm besitzt, **dadurch gekennzeichnet, dass** das Massenverhältnis zwischen dem Xanthangummi und dem Akaziengummi von 4/1 bis 1/1 variiert.

2. Co-Granulat nach Anspruch 1, das einen mittleren Durchmesser zwischen 100 µm und 300 µm, bevorzugt gleich etwa 150 µm besitzt.

3. Co-Granulat nach einem der Ansprüche 1 oder 2, **dadurch gekennzeichnet, dass** höchstens 4 Masse-% der Partikel des Co-Granulats einen mittleren Durchmesser größer als 500 µm haben, und von 65 Masse-% bis 90 Masse-% der Partikel des Co-Granulats einen mittleren Durchmesser größer als 80 µm haben.

4. Co-Granulat nach einem der vorstehenden Ansprüche, das eine Massendichte zwischen 0,3 mg/ml und 0,8 mg/ml und bevorzugt zwischen 0,35 mg/ml und 0,7 mg/ml hat.

5. Co-Granulat nach einem der vorstehenden Ansprüche, **dadurch gekennzeichnet, dass** das Massenverhältnis zwischen dem Xanthangummi und dem Akaziengummi gleich 3/1 ist.

6. Zusammensetzung, umfassend mindestens eine Tablette, die mindestens einen Wirkstoff und/oder mindestens einen Nahrungsstoff enthält, **dadurch gekennzeichnet, dass** die Tablette von 15 Masse-% bis 50 Masse-% Co-Granulate und bevorzugt von 20 % bis 40 %, wie in einem der Ansprüche 1 bis 5 definiert, umfasst.

7. Zusammensetzung nach dem vorstehenden Anspruch, **dadurch gekennzeichnet, dass** die Tablette weiter mindestens einen Hilfsstoff umfasst, der ausgewählt ist aus mindestens: einem Verdünnungsmittel, einem Gleitmittel, einem Kohäsionsmittel.

8. Zusammensetzung nach Anspruch 7, **dadurch gekennzeichnet, dass** sie für 100 % ihrer Masse von 20 Masse-% bis 35 Masse-% Co-Granulate, wie in einem der Ansprüche 1 bis 5 definiert, von 0,1 Masse-% bis 60 Masse-% eines Wirkstoffs und/oder eines Nahrungsstoffs, von 10 Masse-% bis 50 Masse-% Verdünnungs- oder Kohäsionsmittel, von 0,5 Masse-% bis 3 Masse-% Gleitmittel umfasst.

9. Zusammensetzung nach einem der Ansprüche 6 bis 8, **dadurch gekennzeichnet, dass** es eine pharmazeutische, Nahrungsergänzungsmittel- oder Nahrungsmittelzusammensetzung ist, die eine verlängerte Freisetzungswirkung hat.

10. Verfahren zur Herstellung einer Zusammensetzung, wie in einem der Ansprüche 6 bis 9 definiert, das mindestens einen Schritt der direkten Komprimierung eines Gemisches umfasst, das von 15 Gew.-% bis 40 Gew.-% Co-Granulate wie in einem der Ansprüche 1 bis 5 definiert, bevorzugt von 20 % bis 40 %, und mindestens einen Wirkstoff und / oder mindestens einen Nahrungsstoff umfasst.

11. Verfahren nach dem vorstehenden Anspruch, wobei die Zusammensetzung weiter mindestens einen Hilfsstoff umfasst, der ausgewählt ist aus mindestens: einem Verdünnungsmittel, einem Gleitmittel, einem Kohäsionsmittel.

12. Verwendung von mindestens einem Co-Granulat, wie in einem der Ansprüche 1 bis 5 definiert, zur Erzeugung einer pharmazeutischen Zusammensetzung, einer Nahrungsergänzungsmittel- oder Nahrungsmittelzusammensetzung wie in einem der Ansprüche 6 bis 9 definiert.

## Claims

1. Co-granule of xanthan gum and acacia gum having an average diameter of between 50 µm and 1000 µm, **characterised in that** the weight ratio between the xanthan gum and the acacia gum varies from 4/1 to 1/1.

2. Co-granule according to claim 1, having an average diameter of between 100 µm and 300 µm, preferably equal to around 150 µm.

3. Co-granule according to one of claims 1 or 2, **characterised in that** at most 4 % by weight of the particles of the co-granule have an average diameter of greater than 500 µm and from 65 % to 90 % by weight of the particles of the co-granule have an average diameter of greater than 80 µm.

4. Co-granule according to one of the preceding claims having a density of between 0.3 mg/mL and 0.8 mg/mL and preferably between 0.35 mg/mL and 0.7 mg/mL.

5. Co-granule according to one of the preceding claims, **characterised in that** the weight ratio between the xanthan gum and the acacia gum is equal to 3/1.

6. Composition comprising at least one tablet containing at least one active ingredient and/or at least one nutritional agent, **characterised in that** said tablet comprises from 15 % to 50 % by weight of co-granules, and preferably from 20 % to 40 %, such as defined in one of claims 1 to 5.

7. Composition according to the preceding claim, **characterised in that** the tablet further comprises at least one excipient selected from among at least: one diluent, one lubricating agent, one cohesion agent.

8. Composition according to claim 7, **characterised in that** it comprises for 100 % of the weight thereof from 20 % to 35 % by weight of co-granules such as defined in one of claims 1 to 5, from 0.1 % to 60 % by weight of an active ingredient and/or of a nutritional agent, from 10 % to 50 % by weight of diluent or cohesion agent, from 0.5 % to 3 % by weight of lubricating agent.

9. Composition according to one of claims 6 to 8, **characterised in that** it is a pharmaceutical composition, dietary supplement or food having a sustained release effect.

10. Method for preparing a composition such as defined in one of claims 6 to 9 comprising at least one step of direct compression of a mixture comprising from 15 % to 40 % by weight of co-granules such as defined in one of claims 1 to 5, preferably from 20 % to 40 %, and of at least one active ingredient and/or of at least one nutritional agent.

11. Method according to the preceding claim, wherein said composition further comprises at least one excipient selected from among at least: one diluent, one lubricating agent, one cohesion agent.

12. Use of at least one co-granule such as defined in one of claims 1 to 5 for the manufacturing of a pharmaceutical composition, of a dietary supplement composition or food such as defined in one of claims 6 to 9.
